# EUROPÄISCHE PATENTSCHRIFT

(11) **EP 1 470 787 B1**
(45) Veröffentlichungstag und Bekanntmachung des Hinweises auf die Patenterteilung: **31.05.2006**
(21) Anmeldenummer: 03009439.5
(22) Anmeldetag: 25.04.2003
(51) Int. Cl.: A61B 17/15

(54) **Vorrichtung zur Vorbereitung einer Femurkondyle**
Device for preparation of a femoral condyle
Dispositif pour la préparation d'un condyle d'un fémur

(43) Veröffentlichungstag der Anmeldung: 27.10.2004
(73) Patentinhaber: Zimmer GmbH, 8404 Winterthur (CH)
(72) Erfinder: Faoro, Francisco, ch-8002 Zürich (CH); Overes, Tom, 8400 Winterthur (CH)
(74) Vertreter: Manitz, Finsterwald & Partner GbR

(56) Entgegenhaltungen:
- EP-A- 0 809 969
- WO-A-01/85038

## Beschreibung

Die Erfindung betrifft eine Vorrichtung zur Vorbereitung einer Femurkondyle beim Einsetzen von monokondylären Knieimplantaten gemäß dem Oberbegriff des Anspruchs 1. Eine Vorrichtung mit den Merkmalen des Oberbegriffs des Anspruchs 1 ist aus der WO 01/85038 A1 bekannt.

Beim Einsetzen von monokondylären, d.h. einseitigen, Knieprothesen müssen die Kondylen der Tibia und des Femur vorbereitet werden, um Anlageflächen an den Knochen zu schaffen, die eine definierte Lage des Tibia- und Femurimplantats der Knieprothese gewährleisten.

Die Anlageflächen werden durch Wegschneiden von Knochenmaterial an den Kondylen erzeugt. Dabei ist man bestrebt, so wenig Knochenmaterial wie möglich zu entfernen. Ferner muss darauf geachtet werden, dass die Schnittflächen an der Tibia und am Femur richtig relativ zueinander orientiert sind, damit das Tibia- und Femurimplantat in einer der natürlichen Bewegung der restlichen, gesunden Seite des Kniegelenks entsprechenden Weise zusammenwirken kann.

Wenn im Verlauf einer Knieoperation zuerst die Tibia vorbereitet und hierbei ein Tibiaplateau erzeugt wird, auf das später ein Tibiaimplantat aufgesetzt wird, dann kommt es bei der Vorbereitung der entsprechenden Femurkondyle darauf an, dass die an der Femurkondyle zu erzeugenden Schnittflächen korrekt relativ zu den am Tibiaplateau erzeugten Schnittflächen ausgerichtet sind.

Aufgabe der Erfindung ist es, eine Vorrichtung der eingangs genannten Art zu schaffen, mit der auf möglichst einfache und zuverlässige Weise ein Femurimplantat mit möglichst hoher Genauigkeit in der richtigen Lage relativ zur Tibia am Femur implantiert werden kann.

Die Lösung dieser Augbage erfolgt durch die Merkmale des Anspruchs 1.

Durch das Aufspreizen des Knies, d.h. durch Vergrößern des Abstandes zwischen Femur und Tibia, können während der Knieoperation das Femur und die Tibia in ihre natürliche Relativlage gebracht werden, wobei diese natürliche Relativlage anhand der natürlichen Spannung der Bänder vom Operateur ermittelt werden kann.

Die Erfindung ermöglicht es, mittels des mit der Aufspreizeinrichtung gekoppelten Funktionsaufsatzes die zur Vorbereitung der Femurkondyle dienende Schnitt- und Bohrlehre im richtigen Abstand zu dem zuvor erzeugten Tibiaplateau zu positionieren. Erfindungsgemäß kann die Positionierung der Schnitt- und Bohrlehre folglich unter gleichzeitiger

Berücksichtigung der natürlichen Relativlage zwischen Femur und Tibia sowie der Dicke des einzusetzenden Tibiaimplantats erfolgen.

Der Winkel von mehr als 90° zwischen Aufspreizabschnitt und Handhabungsabschnitt hat den Vorteil, dass der Handhabungsabschnitt schräg zur Aufspreizrichtung verläuft und sich also während der Operation vom Knie des Patienten weg erstreckt, wodurch die Handhabung wesentlich erleichtert wird und mehr Platz zur Verfügung steht, was sich bei monokondylären Operationen besonders vorteilhaft auswirkt. Ferner begünstigt dieser erfindungsgemäße Vorrichtungsaufbau in vorteilhafter Weise minimalinvasive Operationstechniken.

Der Winkel α zwischen Aufspreizabschnitt und Handhabungsabschnitt kann z.B. im Bereich von 110° bis 130° liegen.

Vorteilhafte Ausführungsformen der Erfindung sind in den Unteransprüchen, der Beschreibung sowie der Zeichnung angegeben.

Die Aufspreizeinrichtung kann ein am Tibiaplateau abstützbares Unterteil, ein am Unterteil verstellbar geführtes Oberteil und ein verstellbar am Unterteil gehaltenes Betätigungsorgan umfassen, das derart mit dem Unterteil und dem Oberteil zusammenwirkt, dass eine Stellbewegung des Betätigungsorgans in einer Aufspreizbewegung des Oberteils weg von dem Unterteil umsetzbar ist.

Dabei kann vorgesehen sein, dass das Oberteil über das Betätigungsorgan von unten beaufschlagt und in Aufspreizrichtung vom Unterteil weggedrückt werden kann.

Eine besonders einfache Handhabbarkeit der Aufspreizeinrichtung ergibt sich, wenn gemäß einem weiteren Ausführungsbeispiel der Erfindung das Betätigungsorgan schräg zur Aufspreizrichtung und das Oberteil parallel zur Aufspreizrichtung relativ zum Unterteil verstellbar ist.

Das Betätigungsorgan kann an seinem freien Ende mit einer ebenen Druckfläche versehen sein, die parallel zu einer mittels der Druckfläche beaufschlagbaren Unterseite des Oberteils und insbesondere senkrecht zur Aufspreizrichtung verläuft.

In einem besonders bevorzugten Ausführungsbeispiel ist vorgesehen, dass eine parallel zur Aufspreizrichtung verlaufende Führung für das Oberteil und der Bereich, an dem das Betätigungsorgan am Oberteil angreift, senkrecht zur Aufspreizrichtung gegeneinander versetzt sind. Ein versehentliches Verkanten des im Unterteil geführten Oberteils während des Aufspreizvorgang wird hierdurch sicher vermieden.

Das Unterteil und das Oberteil können jeweils eine plattenförmige Spreizzunge aufweisen, mit der sich das Unterteil am Tibiaplateau abstützt und das Oberteil gegen die Femurkondyle drückt.

Ferner kann vorgesehen sein, dass das Betätigungsorgan in den Zwischenraum zwischen den beiden Spreizzungen hinein und gegen die Unterseite der Spreizzunge des Oberteils treibbar ist, wobei das Unterteil einen schräg zu seiner Spreizzunge verlaufenden Basisabschnitt umfasst, in welchem die Führung für das Oberteil ausgebildet und das Betätigungsorgan gehalten ist.

Das Betätigungsorgan kann als Stellschraube ausgebildet sein, so dass eine Schraubbewegung der Stellschraube in eine lineare Aufspreizbewegung umgesetzt wird. Durch entsprechende Wahl des Gewindes kann das Übersetzungsverhältnis grundsätzlich beliebig vorgegeben werden.

Des Weiteren kann erfindungsgemäß vorgesehen sein, dass die Aufspreizeinrichtung mit einer insbesondere in Form einer Skala vorgesehenen Anzeigeeinrichtung versehen ist, mittels welcher eine von der Dicke des einzusetzenden Tibiaimplantats abhängige Sollhöhe des Funktionsaufsatzes an der Aufspreizeinrichtung ablesbar ist.

In Abhängigkeit von der Dicke des gemäß der Operationsplanung einzusetzenden Tibiaimplantats kann auf diese Weise die richtige Höhe des Funktionsaufsatzes und damit der Schnitt- und Bohrlehre exakt eingestellt werden.

Eine vorteilhafte Ausrichtung des Funktionsaufsatzes und damit der Schnitt- und Bohrlehre relativ zum Femur wird ermöglicht, wenn gemäß einem weiteren Ausführungsbeispiel der Erfindung die Aufspreizeinrichtung derart ausgebildet ist, dass sie im zwischen dem Tibiaplateau und der Femurkondyle angeordneten Zustand relativ zum Knie bewegbar ist. Bei den Bewegungsfreiheitsgraden handelt es sich insbesondere um eine Verschiebbarkeit im Wesentlichen senkrecht zur Aufspreizrichtung und/oder um eine Verdrehbarkeit um eine im Wesentlichen parallel zur Aufspreizrichtung verlaufende Achse.

Ferner wird erfindungsgemäß vorgeschlagen, dass die Aufspreizeinrichtung mit mehreren unterschiedlich ausgebildeten Funktionsaufsätzen koppelbar ist. Dies ermöglicht es insbesondere, die Aufspreizeinrichtung sowohl bei in Extension als auch bei in Flexion befindlichem Knie zu verwenden und jeweils entsprechend ausgebildete Funktionsaufsätze einzusetzen.

Wenigstens ein Funktionsaufsatz kann als Schnittlehre zur Festlegung eines bei in Extension befindlichem Knie auszuführenden Kondylenschnittes ausgebildet sein, der insbesondere im Wesentlichen parallel zum Tibiaplateau verläuft.

Ferner kann wenigstens ein Funktionsaufsatz als Schnittlehre zur Festlegung eines bei in Flexion befindlichem Knie auszuführenden Kondylenschnittes ausgebildet sein, der insbesondere im Wesentlichen parallel zum Tibiaplateau verläuft.

Des Weiteren kann vorgesehen sein, dass wenigstens ein Funktionsaufsatz als Bohrlehre zur Festlegung zumindest einer zum Fixieren eines Femurimplantats dienenden, bei in Flexion befindlichem Knie auszuführenden Kondylenbohrung ausgebildet ist, die insbesondere im Wesentlichen parallel zum Tibiaplateau verläuft.

Wenigstens ein Funktionsaufsatz, der insbesondere als kombinierte Schnitt- und Bohrlehre ausgebildet ist, kann an der Femurkondyle fixierbar sein.

Ferner kann wenigstens ein Funktionsaufsatz einen im mit der Aufspreizrichtung gekoppelten Zustand schräg zur Aufspreizrichtung verlaufenden, mit der Aufspreizeinrichtung koppelbaren Körperabschnitt mit einer Feststelleinrichtung und einen fest mit dem Körperabschnitt verbundenen Kopfabschnitt umfassen, der als Schnitt- und/oder Bohrlehre oder als Träger für eine separate Schnitt- und/oder Bohrlehre ausgebildet ist.

Der Kopfabschnitt kann als Schnittlehre mit einem eine Schnittebene definierenden, im mit der Aufspreizeinrichtung gekoppelten Zustand senkrecht zur Aufspreizrichtung verlaufenden Schlitz für ein Schneidewerkzeug ausgebildet sein. Bei dem Schneidewerkzeug handelt es sich insbesondere um das Sägeblatt einer Knochensäge.

Alternativ kann vorgesehen sein, dass der Kopfabschnitt als Träger für die separate kombinierte Schnitt- und Bohrlehre ausgebildet ist, die lösbar mit dem Kopfabschnitt verbindbar und bei in Flexion befindlichem Knie an der Femurkondyle fixierbar ist. Dabei kann die separate kombinierte Schnitt- und Bohrlehre im mit dem Kopfabschnitt verbundenen Zustand längs des Kopfabschnitts verstellbar sein.

Ferner wird erfindungsgemäß vorgeschlagen, dass wenigstens ein Funktionsaufsatz, der insbesondere als kombinierte Schnitt- und Bohrlehre ausgebildet ist, mit einer zusätzlichen Schnittlehre koppelbar ist, die zur Festlegung eines weiteren Kondylenschnittes insbesondere bei in Flexion befindlichem Knie ausgebildet ist, wobei der weitere Kondylenschnitt gekrümmt zwischen zwei zuvor an der Femurkondyle hergestellten ebenen Schnittflächen verläuft.

Die erfindungsgemäße Vorrichtung kann somit in Form eines Multifunktionsinstrumentes vorgesehen sein, das sowohl bei in Extension als auch in Flexion befindlichem Knie verwendbar ist, um eine Schnitt- und/oder Bohrlehre relativ zur vorzubereitenden Femurkondyle zu positionieren.

Von Vorteil ist dies insbesondere dann, wenn Femurimplantate zum Einsatz kommen, die eine konstante Dicke aufweisen. Eine insbesondere als Skala ausgebildete Anzeigeeinrichtung der Aufspreizeinrichtung kann dann sowohl für in Extension als auch für in Flexion auszuführende, im Wesentlichen parallel zum Tibiaplateau verlaufende Femurkondylenschnitte dazu verwendet werden, diese Kondylenschnitte jeweils in Abhängigkeit von der Dicke des einzusetzenden Tibiaimplantats zu setzen.

Die Erfindung wird im Folgenden beispielhaft unter Bezugnahme auf die Zeichnung beschrieben. Es zeigen:
- Fig. 1 bis 5: verschiedene Phasen eines Teils einer Knieoperation, bei dem mittels einer erfindungsgemäßen Vorrichtung eine Femurkondyle zum Einsetzen eines Femurimplantats vorbereitet wird,
- Fig. 6 und 7: jeweils verschiedene Ansichten von Bestandteilen einer Aufspreizeinrichtung gemäß einer Ausführungsform der Erfindung,
- Fig. 8: einen mit der Aufspreizeinrichtung von Fig. 6 und 7 koppelbaren Funktionsaufsatz
- Fig. 9: eine Vorrichtung im zusammengesetzten Zustand,
- Fig. 10: verschiedene Ansichten eines Teils eines mit der Aufspreizrichtung von Fig. 6 und 7 koppelbaren weiteren Funktionsaufsatzes gemäß einer Ausführungsform der Erfindung, und
- Fig. 11: verschiedene Ansichten einer mit dem Funktionsaufsatz-Teil von Fig. 10 koppelbaren kombinierten Schnitt- und Bohrlehre gemäß einer Ausführungsform der Erfindung.

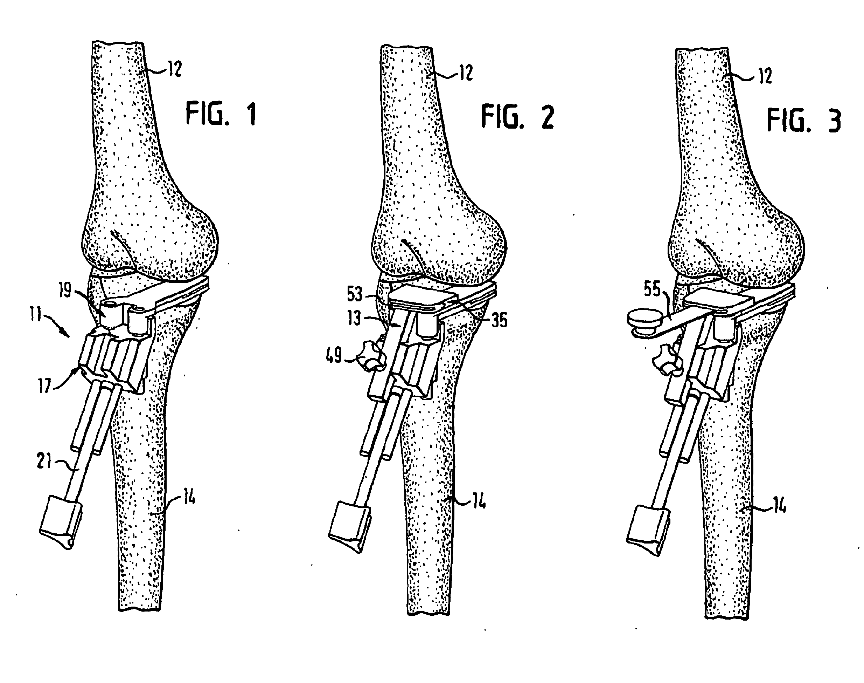

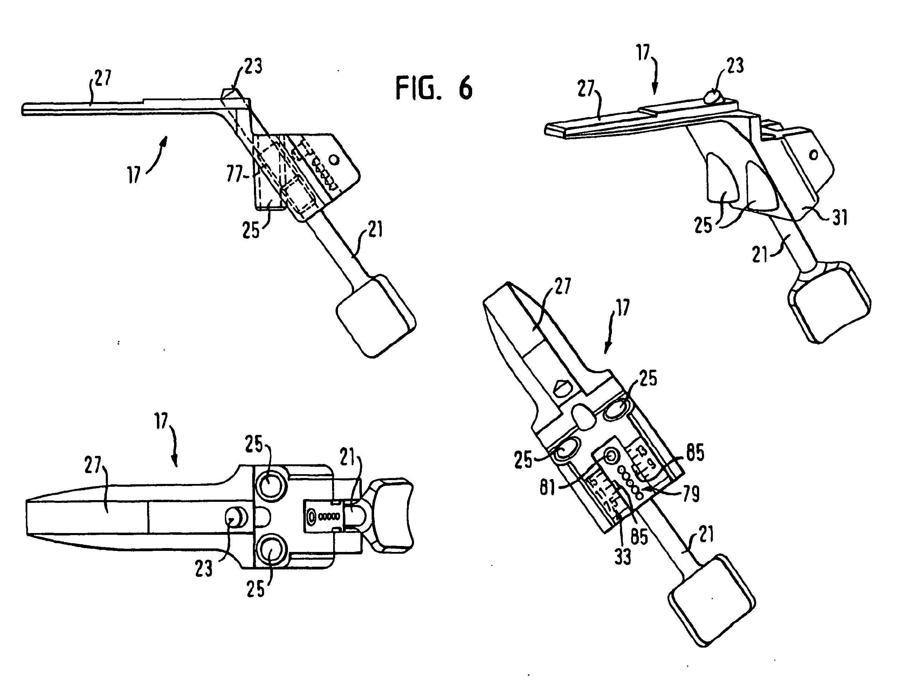

Bevor anhand der Fig. 6 bis 11 auf konstruktive Details der erfindungsgemäßen Vorrichtung eingegangen wird, soll zunächst anhand der Fig. 1 bis 5 derjenige Teil einer Knieoperation erläutert werden, bei dem die erfindungsgemäße Vorrichtung zum Einsatz kommt.

Bei der Operation, in der die erfindungsgemäße Vorrichtung gemäß dem in den Figuren gezeigten Ausführungsbeispiel zum Einsatz kommt, handelt es sich um eine unikompartimentale Operation, bei der entweder im lateralen oder im medialen Kompartiment die Tibiakondyle und die Femurkondyle zum Einsetzen eines Tibiaimplantats bzw. Femurimplantats vorbereitet werden.

Ausgangspunkt für denjenigen Teil der Operation, in dem die erfindungsgemäße Vorrichtung verwendet wird, ist hier ein durch zwei senkrecht zueinander verlaufende Knochenschnitte hergestelltes Tibiaplateau, auf das ein gemäß der Operationsplanung vorgesehenes Tibiaimplantat mit einer bestimmten Dicke gesetzt werden kann, sobald mit Hilfe der erfindungsgemäßen Vorrichtung auch die gegenüberliegende Femurkondyle zur Fixierung eines entsprechenden Femurimplantats vorbereitet ist.

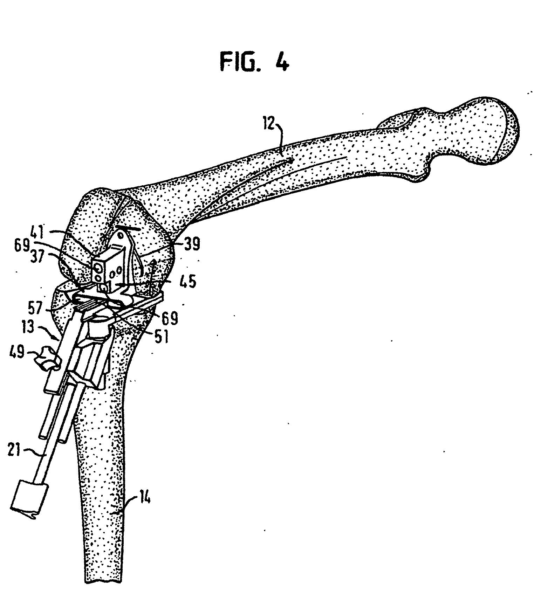

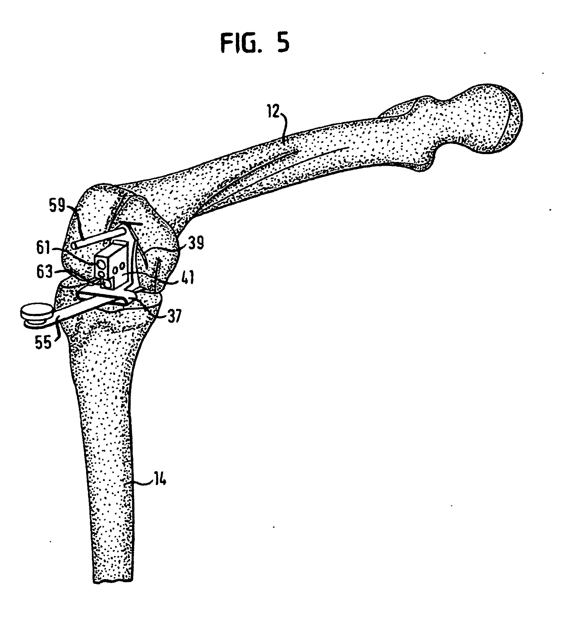

Das erfindungsgemäßen Instrumentarium umfasst gemäß der im Folgenden beschriebenen Ausführungsform eine Aufspreizeinrichtung 11, die mit zwei unterschiedlichen Funktionsaufsätzen 13 koppelbar ist, von denen der eine bei in Extension befindlichem Knie (vgl. Fig. 1 bis 3) und der andere bei in Flexion befindlichem Knie (vgl. Fig. 4 und 5) zum Einsatz kommt.

Die Aufspreizeinrichtung 11 umfasst ein Unterteil 17, an dem ein Oberteil 19 in Aufspreizrichtung verstellbar geführt und ein Betätigungsorgan 21 in Form einer Stellschraube verstellbar gehalten ist. Durch Betätigen der Stellschraube 21 kann das Oberteil 19 nach oben vom Unterteil 17 weg bewegt werden, um das Knie aufzuspreizen, d.h. das Femur 12 von der Tibia 14 und damit die noch vorzubereitende Femurkondyle von dem bereits vorbereiten Tibiaplateau wegzudrücken.

Das Unterteil 17 der Aufspreizeinrichtung 11 stützt sich am zuvor hergestellten Tibiaplateau ab, während das Oberteil 19 direkt die gegenüberliegende Femurkondyle beaufschlagt.

Zunächst wird gemäß Fig. 1 bis 3 das Knie in Extension durch Betätigen der Stellschraube 21 aufgespreizt, bis die natürliche Relativstellung zwischen Femur 12 und Tibia 14 erreicht ist, die der Operateur an der natürlichen Spannung der Bänder erkennen kann.

Anschließend wird ein bereichsweise als mit dem Unterteil 17 der Aufspreizeinrichtung 11 koppelbarer Schlitten ausgebildeter Funktionsaufsatz 13, der ein als Schnittlehre 35 mit einem Schlitz 53 für ein Sägeblatt 55 ausgebildetes Kopfteil aufweist, längs des Unterteils 17 und damit schräg zur Aufspreizrichtung verschoben, bis die von der Dicke des gemäß der Operationsplanung einzusetzenden Tibiaimplantats abhängige richtige Höhe des Schlitzes 53 der Schnittlehre 35 erreicht ist. Hierzu ist am Unterteil 17 eine Skala angebracht, an welcher der Operateur das Erreichen der korrekten Position des Funktionsaufsatzes 13 und damit des Schlitzes 53 ablesen kann. In dieser Position wird der Funktionsaufsatz 13 mittels einer Feststelleinrichtung 49 am Unterteil 17 fixiert.

Anschließend wird mittels eines durch den Schlitz 53 hindurch geführten Sägeblatts 55 ein parallel zum Tibiaplateau verlaufender erster Schnitt in der Femurkondyle ausgeführt, wobei die Schnitttiefe derart gewählt ist, dass die Femurkondyle lediglich eingeschnitten, jedoch kein Teil des Knochens vollständig abgetrennt wird. Es wird also mittels des Sägeblatts 55 zunächst nur ein Schlitz in der Femurkondyle ausgebildet.

Bevor dieser erste Kondylenschnitt ausgeführt wird, kann entweder der Funktionsaufsatz 13 oder die Aufspreizeinrichtung 11 über ein nicht dargestelltes Kontrollgestänge relativ zum Femur 12 ausgerichtet werden, damit der erste Kondylenschnitt nicht nur in der korrekten Höhe, sondern auch bezüglich der Richtung lateral-medial in der richtigen Position ausgeführt wird.

Das Ausführen dieses ersten Femurkondylenschnittes erfolgt bei in seine natürliche Stellung aufgespreiztem Knie. Die Koppelung des Funktionsaufsatzes 13 an das Unterteil 17 kann vor oder nach dem Aufspreizen des Knies erfolgen.

Der zur Vorbereitung der Femurkondyle in Extension dienende Funktionsaufsatz 13 wird anschließend abgenommen und durch einen anderen Funktionsaufsatz 13 ersetzt (vgl. Fig. 4 und 5), der zur Vorbereitung der Femurkondyle bei in Flexion befindlichem Knie dient. In Fig. 4 und 5 ist jetzt der zuvor in Extension ausgeführte erste Kondylenschnitt 39 zu erkennen.

Der Kopfabschnitt 51 des Funktionsabschnitts 13 ist als Träger für eine separate, kombinierte Schnitt- und Bohrlehre 45 ausgebildet, die einen als Schnittlehre 37 und einen als Bohrlehre 41 ausgebildeten Abschnitt umfasst. Die Schnitt- und Bohrlehre 45 entspricht mit ihrem Bohrlehrenabschnitt 41 insofern dem gemäß der Operationsplanung einzusetzenden Femurimplantat, als zum einen die Krümmung der der Femurkondyle zugewandten Seite des Bohrlehrenabschnitts 41 der Krümmung der entsprechenden Femurimplantatseite entspricht und zum anderen im Bohrlehrenabschnitt 41 ausgebildete Durchgänge 69 hinsichtlich Lage und Orientierung bezüglich der gekrümmten Seite den Zapfen des einzusetzenden Femurimplantats entsprechen.

Die kombinierte Schnitt- und Bohrlehre 45 ist hinsichtlich ihres Bohrlehrenabschnitts 41 in verschiedenen Größen vorhanden, die den verschiedenen vorhandenen Femurimplantatgrößen entsprechen.

Des Weiteren ist die kombinierte Schnitt- und Bohrlehre 45 auf dem Kopfabschnitt 51 des Funktionsaufsatzes 13 senkrecht zur Aufspreizrichtung längsverschieblich angeordnet und kann auf diese Weise mit ihrer gekrümmten Seite bis an die Femurkondyle heran geschoben werden. Bevor die Lehre 45 an der Femurkondyle fixiert wird, kann sie mittels einer nicht dargestellten Ausrichthilfe relativ zum Femur 12 ausgerichtet werden. Eine Bewegung der Lehre 45 relativ zum Femur 12 ist dadurch möglich, dass die erfindungsgemäße Aufspreizeinrichtung 11 im zwischen Tibiaplateau und Femurkondyle angeordneten Zustand, d.h. bei aufgespreiztem Knie, relativ zum Knie bewegbar ist, und zwar sowohl im Wesentlichen parallel zum Tibiaplateau und damit senkrecht zur Aufspreizrichtung verschiebbar als auch um eine senkrecht zum Tibiaplateau verlaufende Achse verdrehbar.

Die Fixierung der Lehre 45 an der Femurkondyle erfolgt dadurch, dass die Lehre 45 zunächst mittels eines Positionierstiftes 59 an der Femurkondyle positioniert und anschließend durch die im Bohrlehrenabschnitt 41 ausgebildeten Fixierdurchgänge 69 hindurch die Femurkondyle vorgebohrt wird, woraufhin die Lehre 45 mittels Schrauben 61, 63 an der Femurkondyle befestigt wird.

Dann wird mittels eines durch den im Schnittlehrenabschnitt 37 ausgebildeten Schlitz 57 hindurch geführten Sägeblatts 55 ein zweiter Kondylenschnitt ausgeführt, der im Wesentlichen senkrecht zum ersten Kondylenschnitt 39 verläuft. Mit dem zweiten Kondylenschnitt wird ein Teil des Knochens vollständig abgetrennt.

Die kombinierte Schnitt- und Bohrlehre 45 selbst und ihre Benutzung zur Vorbereitung der Femurkondyle sind nicht Gegenstand der Erfindung, so dass hierauf im Folgenden nicht näher eingegangen wird. Der Vollständigkeit halber sei noch erwähnt, dass zur weitergehenden Femurkondylenvorbereitung die an der Femurkondyle fixierte Lehre 45 mit einer nicht dargestellten zusätzlichen Schnittlehre gekoppelt wird, die dazu dient, einen entsprechend der Krümmung der der Femurkondyle zugewandten Seite der Lehre 45 gekrümmt verlaufenden weiteren Kondylenschnitt auszuführen, der die beiden zuvor hergestellten ebenen Schnittflächen miteinander verbindet.

Wie Fig. 5 zeigt, ist die Bearbeitung der Femurkondyle bei in Flexion befindlichem Knie ohne die erfindungsgemäße Aufspreizeinrichtung 11 möglich, die abgenommen werden kann, sobald die kombinierte Schnitt- und Bohrlehre 45 an der Femurkondyle fixiert ist. Es ist jedoch die erfindungsgemäße Aufspreizvorrichtung 11, die eine korrekte Positionierung sowohl in Extension als auch in Flexion des jeweiligen Funktionsabschnitts 13 und damit der jeweiligen Lehre 35, 45 gestattet, wobei dies bei aufgespreiztem und damit seine natürliche Stellung einnehmenden Knie erfolgt.

Gemäß Fig. 6 umfasst das Unterteil 17 der erfindungsgemäßen Aufspreizeinrichtung 11 eine Spreizzunge 27, mit der sich die Aufspreizeinrichtung 11 am Tibiaplateau abstützen kann. Schräg zur Aufspreizzunge 27 erstreckt sich ein Basisabschnitt 31 des Unterteils 17, in dem zwei gemeinsam als Führung 25 für das in Fig. 6 nicht dargestellte Oberteil dienende Durchgänge ausgebildet sind, die senkrecht zur Spreizzunge 27 verlaufen.

Eine als Betätigungsorgan für die Aufspreizeinrichtung 11 dienende Stellschraube 21 erstreckt sich durch den Basisabschnitt 31 parallel zu diesem hindurch. Der Basisabschnitt 31 und die Stellschraube 21 wirken über ein Gewinde 77 zusammen, so dass durch Drehen an der Stellschraube 21 eine am freien Ende der Stellschraube 21 ausgebildete, parallel zur Spreizzunge 27 verlaufende Druckfläche 23 relativ zur Spreizzunge 27 in der Höhe verstellbar ist.

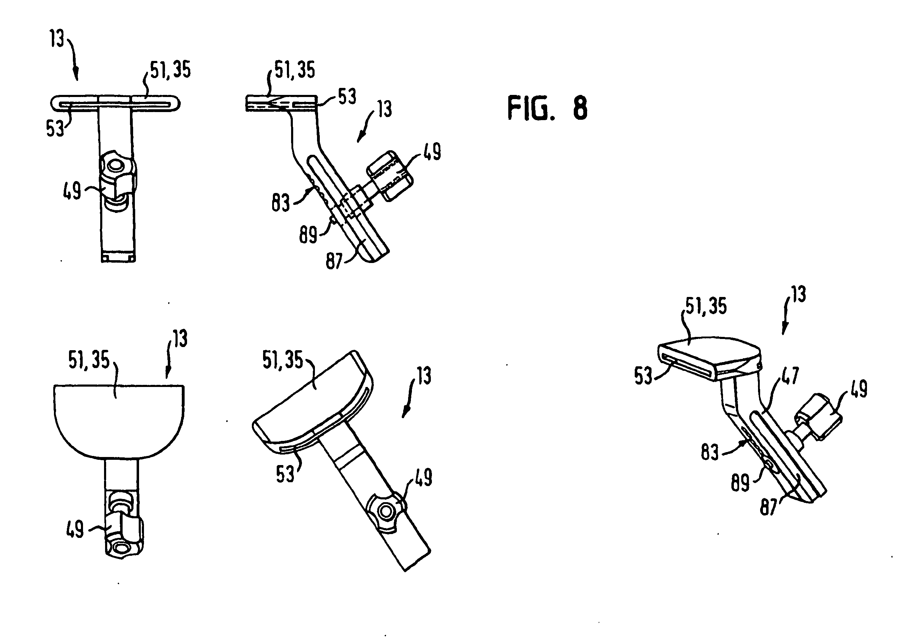

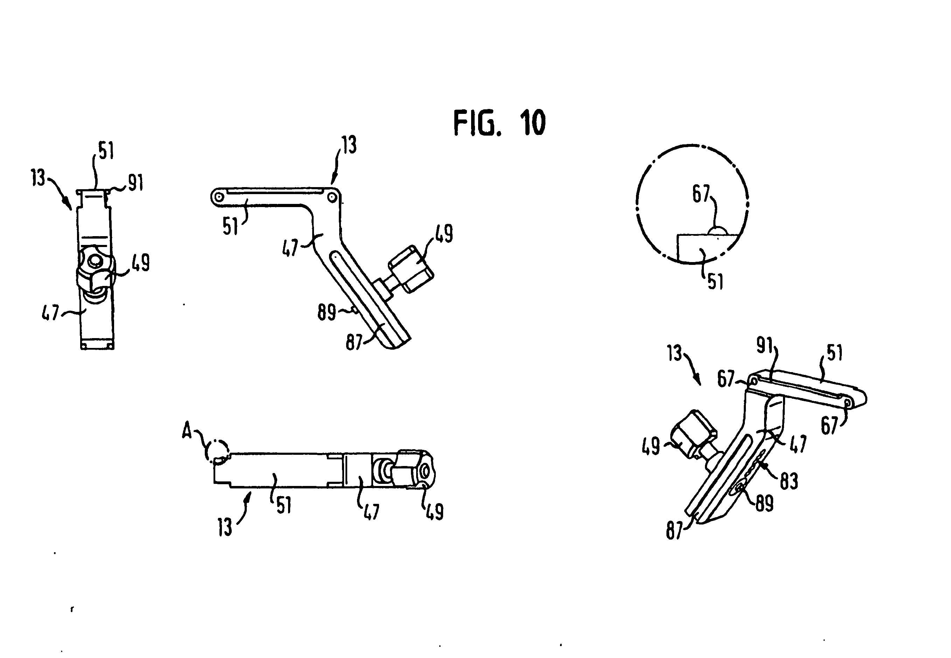

Der außerdem zur Koppelung mit den Funktionsaufsätzen 13 ausgebildete Basisabschnitt 31 des Unterteils weist eine Reihe 79 von Feststellaussparungen auf, mit denen die Feststelleinrichtung 49 des jeweiligen Funktionsaufsatzes (vgl. Fig. 8 und 10) in Eingriff bringbar ist, um den Funktionsaufsatz 13 am Unterteil 17 festzusetzen. Ein federbelastetes Rastorgan 81 des Unterteils 17 wirkt jeweils mit einer Reihe 83 von an den Funktionsaufsätzen 13 ausgebildeten Rastaussparungen zusammen. Seitliche Führungsvorsprünge 85 des Unterteils 17 greifen in seitliche Nuten 87 der Funktionsaufsätze 13 ein, wodurch beim Verstellen der Funktionsaufsätze 13 relativ zur Aufspreizeinrichtung 11 eine Zwangsführung für die Funktionsaufsätze 13 erzielt wird.

Die Funktionsaufsätze 13 sind folglich nicht stufenlos an dem Unterteil 17 der Aufspreizeinrichtung 11 verstellbar, sondern es können lediglich die durch die erwähnten Feststell- und Verrastungsmittel vorgegebenen diskreten Konfigurationen eingestellt werden, die den unterschiedlichen Dicken der zur Verfügung stehenden Tibiaimplantate entsprechen.

Die in diesem Ausführungsbeispiel beschriebene Aufspreizeinrichtung wird in Verbindung mit solchen Implantatsätzen verwendet, bei denen lediglich die Dicke der Tibiaimplantate variiert, die Dicke der Femurimplantate dagegen konstant ist. Das Einstellen der Position der Funktionsaufsätze 13 relativ zur Aufspreizeinrichtung 11 und damit der Höhe der jeweiligen Schnittlehre 35 bzw. 37 über dem Tibiaplateau erfolgt somit lediglich in Abhängigkeit von der Dicke des jeweils gemäß der Operationsplanung vorgesehenen Tibiaimplantats. Diese Dicke kann bei mit der Aufspreizeinrichtung 11 gekoppeltem Funktionsaufsatz 13 an einer am Unterteil 17 ausgebildeten Skala 33 abgelesen werden.

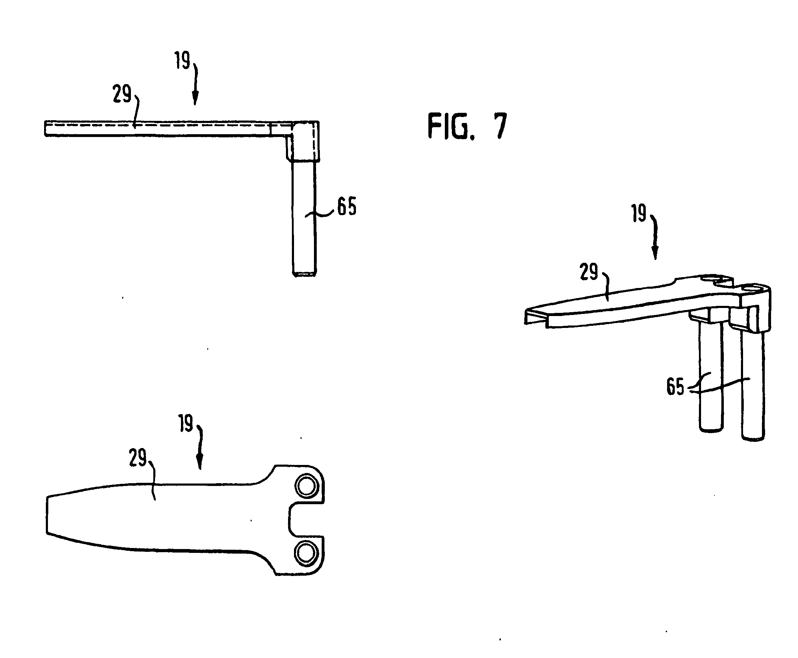

Das in Fig. 7 dargestellte Oberteil 19 der Aufspreizeinrichtung 11 umfasst eine Spreizzunge 29, die senkrecht zu zwei Führungsstangen 65 verläuft, mit denen das Oberteil 19 in die Führungsdurchgänge 25 des Unterteils 17 gesteckt wird.

Fig. 8 zeigt den in Extension verwendeten Funktionsaufsatz 13, dessen Kopfabschnitt 51 als mit einem Schlitz 53 für ein Sägeblatt versehene Schnittlehre 35 zur Ausführung des ersten Kondylenschnittes (vgl. Fig. 3) ausgebildet ist.

An einem sich schräg zum Schlitz 53 erstreckenden Körperabschnitt 47 des Funktionsaufsatzes 13 sind die seitlichen Führungsnuten 87 und die Reihe von Rastaussparungen 83 ausgebildet. Ferner ist an dem Körperabschnitt 47 die Feststelleinrichtung 49 angebracht, die mit dem freien Ende eines Feststellstiftes 89 in die am Unterteil 17 der Aufspreizeinrichtung 11 ausgebildeten Feststellaussparungen 79 eingreift, wenn die Feststelleinrichtung 49 entsprechend betätigt wird, um den Funktionsaufsatz 13 an der Aufspreizeinrichtung 11 festzusetzen.

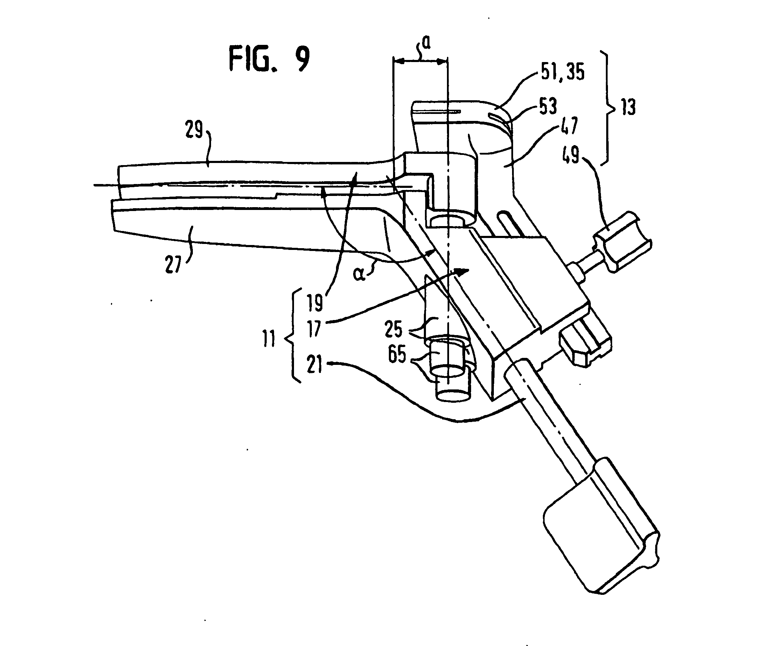

Fig. 9 zeigt die erfindungsgemäße Vorrichtung im für die Verwendung bei in Extension befindlichem Knie zusammengesetzten Zustand, d.h. mit dem Funktionsaufsatz 13 gemäß Fig. 8 zur Ausführung des ersten Kondylenschnittes 39.

Die Spreizzungen 27, 29 des Unterteils 17 und des Oberteils 19 verlaufen parallel zueinander. Der Bereich, an dem die Druckfläche 23 (vgl. Fig. 6) des Betätigungsorgans 21 die Unterseite der Spreizzunge 29 des Oberteils 19 beaufschlagt, ist senkrecht zur Aufspreizrichtung, d.h. parallel zu von den Spreizzungen 27, 29 festgelegten Ebenen, gegenüber der durch die Führungsdurchgänge 25 des Unterteils 17 festgelegten, parallel zur Aufspreizrichtung verlaufenden Führungsachse für jede Stellung des Betätigungsorgans 21 um einen von Null verschiedenen Betrag a versetzt, wie es in Fig. 9 angedeutet ist. Hierdurch kommt es zu keinem Verkanten der Führungsstangen 65 des Oberteils 19 in den Führungsdurchgängen 25 des Unterteils 17, wenn die Stellschraube 49 betätigt wird.

Des Weiteren geht aus Fig. 9 der vorteilhafte Aufbau der erfindungsgemäßen Vorrichtung mit dem 90° übersteigenden Winkel α zwischen dem die Spreizzungen 27, 29 umfassenden Aufspreizabschnitt und dem den Basisabschnitt 31 und das Betätigungsorgan 21 umfassenden Handhabungsabschnitt hervor.

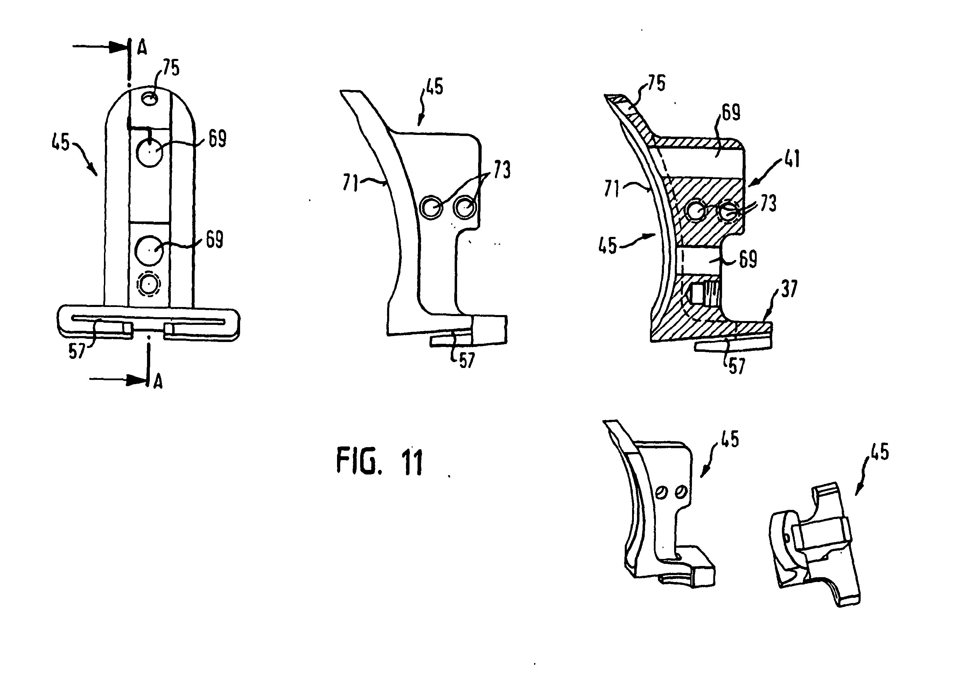

Bei dem in Fig. 10 dargestellten, zur Vorbereitung der Femurkondyle bei in Flexion befindlichem Knie (vgl. Fig. 4 und 5) verwendeten Funktionsaufsatz 13 ist der Kopfabschnitt 51 als Träger für die in Fig. 11 gezeigte kombinierte Schnitt- und Bohrlehre 45 ausgebildet. Der Kopfabschnitt 51 weist eine im Querschnitt T-förmige Kopplungsschiene 91 auf, auf welche die Lehre 45 über einen als Gleitsteckschuh ausgebildeten Abschnitt aufgeschoben werden kann. Als überwindbare Anschläge dienen federbelastete Rastköpfe 67, die jeweils zu beiden Seiten an den Enden der Kopplungsschiene 91 des Kopfteils 51 angebracht sind.

Die in verschiedenen, einem Satz von vorhandenen Femurimplantaten entsprechenden Größen vorgesehene kombinierte Schnitt- und Bohrlehre 45 ist im unteren Bereich als mit einem Schlitz 57 für ein Sägeblatt versehene Schnittlehre 37 und im oberen Bereich als mit zwei Fixierdurchgängen 69 versehene Bohrlehre 41 ausgebildet, durch die jeweils ein Bohrer zum Vorbohren des Knochens und anschließend ein Fixierelement, insbesondere eine Knochenschraube, zum Fixieren der Lehre 45 am Knochen hindurchführbar sind.

Wie bereits vorstehend erwähnt, entspricht die Lehre 45 hinsichtlich der Krümmung ihrer während der Operation der Femurkondyle zugewandten konkaven Seite 71 sowie hinsichtlich der Lage und der Orientierung der Fixierdurchgänge 69 bezüglich der gekrümmten Seite 71 einem Femurimplantat gleicher Größe. Insofern wird bei der Operation mittels der Lehre 45 das einzusetzende Femurimplantat simuliert.

An ihrem oberen Ende ist die Lehre 45 mit einem Positionierdurchgang 75 versehen, durch den der Positionierstift 59 (vgl. z.B. Fig. 5) hindurchgeführt wird, um die Lehre 45 an der Femurkondyle zu halten, während über die Fixierdurchgänge 69 in der Femurkondyle die Bohrungen für die Fixierschrauben 61, 63 bzw. für die Zapfen des einzusetzenden Femurimplantats ausgeführt werden.

Des Weiteren ist die Lehre 45 in dem Bereich zwischen den beiden Fixierdurchgängen 69 mit quer verlaufenden Kopplungsdurchgängen 73 versehen, die gemeinsam als Kopplungsabschnitt dienen, über den die Lehre 45 mit weiteren Instrumenten, insbesondere mit Ausrichthilfen, Manipulationseinrichtungen und weiteren Schnittlehren, gekoppelt werden kann.

### Bezugszeichenliste

- 11: Aufspreizeinrichtung
- 12: Femur
- 13: Funktionsaufsatz
- 14: Tibia
- 17: Unterteil
- 19: Oberteil
- 21: Betätigungsorgan, Stellschraube
- 23: Druckfläche
- 25: Führung
- 27: Spreizzunge des Unterteils
- 29: Spreizzunge des Oberteils
- 31: Basisabschnitt des Unterteils
- 33: Anzeigeeinrichtung, Skala
- 35: Schnittlehre für Extension
- 37: Schnittlehre für Flexion
- 39: erster Kondylenschnitt, erste Schnittfläche
- 41: Bohrlehre für Flexion
- 45: kombinierte Schnitt- und Bohrlehre
- 47: Körperabschnitt des Funktionsaufsatzes
- 49: Feststelleinrichtung
- 51: Kopfabschnitt des Funktionsaufsatzes
- 53: Schlitz für Extension
- 55: Schneidewerkzeug, Sägeblatt
- 57: Schlitz für Flexion
- 59: Positionierstift
- 61: Fixierelement, Schraube
- 63: Fixierelement, Schraube
- 65: Führungsstange
- 67: Rastkopf
- 69: Fixierdurchgang
- 71: gekrümmte Seite
- 73: Kopplungsdurchgang
- 75: Positionierdurchgang
- 77: Gewinde
- 79: Reihe von Feststellaussparungen
- 81: Rastorgan
- 83: Reihe von Rastaussparungen
- 85: Führungsvorsprung
- 87: seitliche Führungsnut
- 89: Feststellstift
- 91: Kopplungsschiene

- a: Versatz
- α: Winkel zwischen Aufspreizabschnitt und Handhabungsabschnitt

## Patentansprüche

1. Vorrichtung zur Vorbereitung einer Femurkondyle beim Einsetzen von monokondylären Knieimplantaten, mit
- einer Aufspreizeinrichtung (11) zum Einstellen eines Sollabstandes zwischen einer Femurkondyle und einem gegenüberliegenden Tibiaplateau, und
- wenigstens einem mit der Aufspreizeinrichtung (11) höhenverstellbar koppelbaren Funktionsaufsatz (13),
wobei
- die Aufspreizeinrichtung (11) einen zwischen Femurkondyle und Tibiaplateau einbringbaren, sich im Wesentlichen senkrecht zur Aufspreizrichtung erstreckenden Aufspreizabschnitt (27, 29) und einen Handhabungsabschnitt (21, 31) für den Aufspreizabschnitt (27) umfasst, der mit dem Aufspreizabschnitt (27, 29) einen Winkel α > 90° einschließt, und
- der Funktionsaufsatz (13) eine kombinierte Schnitt- und Bohrlehre (45) trägt und bei mittels der Aufspreizeinrichtung (11) eingestelltem Sollabstand am Handhabungsabschnitt (21, 31) relativ zur Aufspreizeinrichtung (11) zumindest in der Höhe verstellbar ist,
**dadurch gekennzeichnet ,**
**dass** kombinierte Schnitt- und Bohrlehren (45) zur Festlegung eines Kondylenschnittes und zumindest einer Kondylenbohrung bei in Flexion befindlichem Knie in verschiedenen, einem Satz von Femurimplantaten entsprechenden Größen vorgesehen sind.

2. Vorrichtung nach Anspruch 1,
**dadurch gekennzeichnet,**
**dass** die zur Festlegung eines Kondylenschnitts und zumindest einer Kondylenbohrung bei in Flexion befindlichem Knie ausgebildeten kombinierten Schnitt- und Bohrlehren (45) jeweils eine konkav gekrümmte Seite (71) aufweisen, deren Krümmung einer entsprechenden Femurimplantatseite entspricht.

3. Vorrichtung nach Anspruch 1 oder 2,
**dadurch gekennzeichnet,**
**dass** die Aufspreizeinrichtung (11) ein am Tibiaplateau abstützbares Unterteil (17), ein am Unterteil (17) verstellbar geführtes Oberteil (19) und ein verstellbar am Unterteil (17) gehaltenes Betätigungsorgan (21) umfasst, das derart mit dem Unterteil (17) und dem Oberteil (19) zusammenwirkt, dass eine Stellbewegung des Betätigungsorgans (21) in eine Aufspreizbewegung des Oberteils (19) weg von dem Unterteil (17) umsetzbar ist.

4. Vorrichtung nach Anspruch 3,
**dadurch gekennzeichnet,**
**dass** das Oberteil (19) über das Betätigungsorgan (21) von unten beaufschlagbar und in Aufspreizrichtung vom Unterteil (17) wegdrückbar ist.

5. Vorrichtung nach Anspruch 3 oder 4,
**dadurch gekennzeichnet,**
**dass** das Betätigungsorgan (21) schräg zur Aufspreizrichtung und das Oberteil (19) parallel zur Aufspreizrichtung relativ zum Unterteil (17) verstellbar ist.

6. Vorrichtung nach einem der Ansprüche 3 bis 5,
**dadurch gekennzeichnet,**
**dass** das Betätigungsorgan (21) an seinem freien Ende mit einer ebenen Druckfläche (23) versehen ist, die parallel zu einer mittels der Druckfläche (23) beaufschlagbaren Unterseite des Oberteils (19) und insbesondere senkrecht zur Aufspreizrichtung verläuft.

7. Vorrichtung nach einem der Ansprüche 3 bis 6,
**dadurch gekennzeichnet,**
**dass** eine parallel zur Aufspreizrichtung verlaufende Führung (25) für das Oberteil (19) und der Bereich, an dem das Betätigungsorgan (21) am Oberteil (19) angreift, senkrecht zur Aufspreizrichtung gegeneinander versetzt sind.

8. Vorrichtung nach einem der Ansprüche 3 bis 7,
**dadurch gekennzeichnet,**
**dass** das Unterteil (17) und das Oberteil (19) jeweils eine plattenförmige Spreizzunge (27, 29) aufweisen, mit der das Unterteil (17) am Tibiaplateau abstützbar und das Oberteil (19) gegen die Femurkondyle drückbar ist.

9. Vorrichtung nach Anspruch 8,
**dadurch gekennzeichnet,**
**dass** das Betätigungsorgan (21) in den Zwischenraum zwischen den beiden Spreizzungen (27, 29) hinein und gegen die Unterseite der Spreizzunge (29) des Oberteils (19) treibbar ist, wobei das Unterteil (17) einen schräg zu seiner Spreizzunge (27) verlaufenden Basisabschnitt (31) umfasst, in welchem eine Führung (25) für das Oberteil (19) ausgebildet und das Betätigungsorgan (21) gehalten ist.

10. Vorrichtung nach einem der Ansprüche 3 bis 9,
**dadurch gekennzeichnet,**
**dass** das Betätigungsorgan (21) als Stellschraube ausgebildet ist.

11. Vorrichtung nach einem der vorhergehenden Ansprüche,
**dadurch gekennzeichnet,**
**dass** die Aufspreizeinrichtung (11) mit einer insbesondere in Form einer Skala vorgesehenen Anzeigeeinrichtung (33) versehen ist, mittels welcher eine von der Dicke des einzusetzenden Tibiaimplantats abhängige Sollhöhe des Funktionsaufsatzes (13) an der Aufspreizeinrichtung (11) ablesbar ist.

12. Vorrichtung nach einem der vorhergehenden Ansprüche,
**dadurch gekennzeichnet,**
**dass** der Funktionsaufsatz (13) zwischen diskreten Stellungen relativ zur Aufspreizeinrichtung (11) verstellbar ist, die entsprechend den Dicken eines Satzes von Tibiaimplantaten unterschiedlicher Dicke beabstandet sind.

13. Vorrichtung nach einem der vorhergehenden Ansprüche,
**dadurch gekennzeichnet,**
**dass** die Aufspreizeinrichtung (11) im zwischen dem Tibiaplateau und der Femurkondyle angeordneten Zustand relativ zum Knie bewegbar ist, insbesondere im Wesentlichen senkrecht zur Aufspreizrichtung verschiebbar und/oder um eine im Wesentlichen parallel zur Aufspreizrichtung verlaufende Achse verdrehbar.

14. Vorrichtung nach einem der vorhergehenden Ansprüche,
**dadurch gekennzeichnet,**
**dass** die Aufspreizeinrichtung (11) mit mehreren unterschiedlich ausgebildeten Funktionsaufsätzen (13) koppelbar ist.

15. Vorrichtung nach einem der vorhergehenden Ansprüche,
**dadurch gekennzeichnet,**
**dass** wenigstens ein Funktionsaufsatz (13) als Schnittlehre (35) zur Festlegung eines bei in Extension befindlichem Knie auszuführenden Kondylenschnittes (39) ausgebildet ist, der insbesondere im Wesentlichen parallel zum Tibiaplateau verläuft.

16. Vorrichtung nach einem der vorhergehenden Ansprüche,
**dadurch gekennzeichnet,**
**dass** wenigstens ein Funktionsaufsatz (13) als Schnittlehre (37) zur Festlegung eines bei in Flexion befindlichem Knie auszuführenden Kondylenschnittes ausgebildet ist, der insbesondere im Wesentlichen parallel zum Tibiaplateau verläuft.

17. Vorrichtung nach einem der vorhergehenden Ansprüche,
**dadurch gekennzeichnet,**
**dass** wenigstens ein Funktionsaufsatz (13) als Bohrlehre (41) zur Festlegung zumindest einer zum Fixieren eines Femurimplantats dienenden, bei in Flexion befindlichem Knie auszuführenden Kondylenbohrung ausgebildet ist, die insbesondere im Wesentlichen parallel zum Tibiaplateau verläuft.

18. Vorrichtung nach einem der vorhergehenden Ansprüche,
**dadurch gekennzeichnet,**
**dass** wenigstens ein Funktionsaufsatz (13), der insbesondere als kombinierte Schnitt- und Bohrlehre ausgebildet ist, an der Femurkondyle fixierbar ist.

19. Vorrichtung nach einem der vorhergehenden Ansprüche,
**dadurch gekennzeichnet,**
**dass** wenigstens ein Funktionsaufsatz (13) einen im mit der Aufspreizeinrichtung (11) gekoppelten Zustand schräg zur Aufspreizrichtung verlaufenden, mit der Aufspreizeinrichtung (11) koppelbaren Körperabschnitt (47) mit einer Feststelleinrichtung (49) und einen fest mit dem Körperabschnitt (47) verbundenen Kopfabschnitt (51) umfasst, der als Schnitt- und/oder Bohrlehre (35) oder als Träger für eine separate Schnitt- und/oder Bohrlehre (45) ausgebildet ist.

20. Vorrichtung nach Anspruch 19,
**dadurch gekennzeichnet,**
**dass** der Kopfabschnitt (51) als Schnittlehre (35) mit einem eine Schnittebene definierenden, im mit der Aufspreizeinrichtung (11) gekoppelten Zustand senkrecht zur Aufspreizrichtung verlaufenden Schlitz (53) für ein Schneidewerkzeug (55) ausgebildet ist.

21. Vorrichtung nach Anspruch 19,
**dadurch gekennzeichnet,**
**dass** der Kopfabschnitt (51) als Träger für die separate kombinierte Schnitt- und Bohrlehre (45) ausgebildet ist, die lösbar mit dem Kopfabschnitt (51) verbindbar und bei in Flexion befindlichem Knie an der Femurkondyle fixierbar ist.

22. Vorrichtung nach Anspruch 21,
**dadurch gekennzeichnet,**
**dass** die separate kombinierte Schnitt- und Bohrlehre (45) im mit dem Kopfabschnitt (51) verbundenen Zustand längs des Kopfabschnitts (51) verstellbar ist.

23. Vorrichtung nach einem der vorhergehenden Ansprüche,
**dadurch gekennzeichnet,**
**dass** eine zusätzliche Schnittlehre vorgesehen ist, und dass wenigstens ein Funktionsaufsatz (13), der insbesondere als kombinierte Schnitt- und Bohrlehre ausgebildet ist, mit der zusätzlichen Schnittlehre koppelbar ist, die zur Festlegung eines weiteren Kondylenschnittes insbesondere bei in Flexion befindlichem Knie ausgebildet ist, wobei der weitere Kondylenschnitt gekrümmt zwischen zwei zuvor an der Femurkondyle hergestellten
ebenen Schnittflächen (39) verläuft.

## Claims

1. An apparatus for the preparation of a femoral condyle for the insertion of monocondylar knee implants, comprising
- a spreading device (11) for the setting of a desired spacing between a femoral condyle and an oppositely disposed tibia plateau; and
- at least one functional attachment (13) couplable in a vertically adjustable manner to the spreading device (11),
wherein
- the spreading device (11) includes a spreading section (27, 29), which can be introduced between the femoral condyle and the tibia plateau and which extends substantially perpendicular to the spreading direction, and a handling section (21, 31) for the spreading section (27) which includes an angle α > 90° with the spreading section (27, 29); and
- the functional attachment (13) supports a cutting and/or drilling jig (35, 45) and is adjustable at least vertically relative to the spreading device (11) when the desired spacing is set at the handling section (21, 31) by means of the spreading device (11),
**characterized in that**
combined cutting and drilling jigs (45) are provided in different sizes corresponding to a set of femur implants for the fixing of a condylar cut and of at least one condylar bore with a knee in flexion.

2. An apparatus in accordance with claim 1, **characterized in that** the combined cutting and drilling jigs (45) made for the fixing of a condylar cut and at least one condylar bore with a knee in flexion each have a concavely curved side (71) whose curvature corresponds to a corresponding side of a femur implant.

3. An apparatus in accordance with claim 1 or claim 2, **characterized in that** the spreading device (11) includes a lower part (17) supportable at the tibia plateau, an upper part (19) adjustably guided at the lower part (17) and an actuation member (21) adjustably held at the lower part (17) which cooperates with the lower part (17) and the upper part (19) such that a setting movement of the actuation member (21) can be translated into a spreading movement of the upper part (19) away from the lower part (17).

4. An apparatus in accordance with claim 3, **characterized in that** the upper part (19) is able to be acted on from below via the actuation member (21) and can be pressed away from the lower part (17) in the spreading direction.

5. An apparatus in accordance with claim 3 or claim 4, **characterized in that** the actuation member (21) is adjustable obliquely to the spreading direction and the upper part (19) is adjustable parallel to the spreading direction relative to the lower part (17).

6. An apparatus in accordance with any one of the claims 3 to 5, **characterized in that** the actuation member (21) is provided at its free end with a planar pressure surface (23) which extends parallel to a lower side of the upper part (19) which can be acted on by means of the pressure surface (23) and extends in particular perpendicular to the spreading direction.

7. An apparatus in accordance with any one of the claims 3 to 6, **characterized in that** a guide (25) for the upper part (19) extending parallel to the spreading direction and the region at which the actuation member (21) acts on the upper part (19) are offset with respect to one another perpendicular to the spreading direction.

8. An apparatus in accordance with any one of the claims 3 to 7, **characterized in that** the lower part (17) and the upper part (19) each have a plate-shaped spreading tongue (27, 29) with which the lower part (17) is supportable at the tibia plateau and the upper part (19) can be pressed toward the femoral condyle.

9. An apparatus in accordance with claim 8, **characterized in that** the actuation member (21) is drivable into the intermediate space between the two spreading tongues (27, 29) and toward the lower side of the spreading tongue (29) of the upper part (19), with the lower part (17) including a base section (31) which extends obliquely to its spreading tongue (27) and in which the guide (25) for the upper part (19) is formed and the actuation member (21) is held.

10. An apparatus in accordance with any one of the claims 3 to 9, **characterized in that** the actuation member (21) is made as an adjustable screw.

11. An apparatus in accordance with any one of the preceding claims, **characterized in that** the spreading device (11) is provided with a display device (33), in particular in the form of a scale, by means of which a desired height of the functional attachment (13) dependent on the thickness of the tibia implant to be inserted can be read off at the spreading device (11).

12. An apparatus in accordance with any one of the preceding claims, **characterized in that** the functional attachment (13) is adjustable between discrete positions relative to the spreading device (11) which are spaced apart corresponding to the thicknesses of a set of tibia implants of different thickness.

13. An apparatus in accordance with any one of the preceding claims, **characterized in that** the spreading device (11) is movable relative to the knee in the condition arranged between the tibia plateau and
the femoral condyle, in particular substantially displaceable perpendicular to the spreading direction and/or rotatable about an axis extending substantially parallel to the spreading direction.

14. An apparatus in accordance with any one of the preceding claims, **characterized in that** the spreading device (11) is couplable to a plurality of differently formed functional attachments (13).

15. An apparatus in accordance with any one of the preceding claims, **characterized in that** at least one functional attachment (13) is made as a cutting jig (35) for fixing the position of a condylar cut (39) which is to be carried out when the knee is in extension and which in particular extends substantially parallel to the tibia plateau.

16. An apparatus in accordance with any one of the preceding claims, **characterized in that** at least one functional attachment (13) is made as a cutting jig (37) for fixing the position of a condylar cut which is to be carried out when the knee is in flexion and which in particular extends substantially parallel to the tibia plateau.

17. An apparatus in accordance with any one of the preceding claims, **characterized in that** at least one functional attachment (13) is made as a drilling jig (41) for fixing the position of at least one condylar bore which serves for the fixing of a femur implant, which is to be carried out when the knee is in flexion and in particular extends substantially parallel to the tibia plateau.

18. An apparatus in accordance with any one of the preceding claims, **characterized in that** at least one functional attachment (13), which is in particular formed as a combined cutting and drilling jig (45), can be fixed to the femoral condyle.

19. An apparatus in accordance with any one of the preceding claims, **characterized in that** at least one functional attachment (13) includes a body section (47) extending obliquely to the spreading direction in the state coupled to the spreading device (11), couplable to the spreading device (11) and having a fixing device (49) and a head section (51) fixedly connected to the body section (47) which is formed as a cutting and/or as a drilling jig (35) or as a support for a separate cutting and/or drilling jig (45).

20. An apparatus in accordance with claim 19, **characterized in that** the head section (51) is made as a cutting jig (35) with a slot (53) for a cutting tool (55) defining a cutting plane and extending perpendicular to the spreading direction in the state coupled to the spreading device (11).

21. An apparatus in accordance with claim 19, **characterized in that** the head section (51) is made as a support for the separate cutting and/or drilling jig (45) which can be releasably connected to the head section (51) and which can be fixed to the femoral condyle when the knee is in flexion.

22. An apparatus in accordance with claim 21, **characterized in that** the cutting and/or drilling jig (45) is adjustable along the head section (51) in the state connected to the head section (51).

23. An apparatus in accordance with any one of the preceding claims, **characterized in that** an additional cutting jig is provided; and **in that** at least one functional attachment (13), which is in particular made as a combined cutting and drilling jig (45) can be coupled to the additional cutting jig which is made for fixing the position of a further condylar cut, in particular when the knee is in flexion, with the further condylar section extending in a curved manner between two planar cut surfaces (39) previously made at the femoral condyle.

## Revendications

1. Dispositif pour la préparation d'un condyle de fémur lors de la mise en place d'implants de genou mono-condyliens, comportant
- un moyen d'écartement (11) pour régler une distance de consigne entre un condyle de fémur et un plateau tibial opposé, et
- au moins une garniture fonctionnelle (13) susceptible d'être couplée avec réglage en hauteur au moyen d'écartement (11),
dans lequel
- le moyen d'écartement (11) comprend un tronçon d'écartement (27, 29) qui est susceptible d'être introduit entre le condyle de fémur et le plateau tibial et qui s'étend sensiblement perpendiculairement à la direction d'écartement, et un tronçon de manipulation (21, 31) pour le tronçon d'écartement (27), qui définit un angle α > 90° avec le tronçon d'écartement (27, 29), et
- la garniture fonctionnelle (13) porte un gabarit de coupe et de perçage combiné (45) et est réglable au moins en hauteur sur le tronçon de manipulation (21, 31) par rapport au moyen d'écartement (11), la distance de consigne étant réglée à l'aide du moyen d'écartement (11),
**caractérisé en ce que**
il est prévu des gabarits de coupe et de perçage combinés (45) pour déterminer une coupe de condyle et au moins un perçage de condyle, le genou étant en flexion, dans différentes tailles correspondant à un lot d'implants fémoraux.

2. Dispositif selon la revendication 1,
**caractérisé en ce que**
les gabarits de coupe et de perçage combinés (45) réalisés pour déterminer une coupe de condyle et au moins un perçage de condyle, le genou étant en flexion, comprennent un côté respectif à courbure concave (71) dont la courbure correspond à un côté d'implant fémoral correspondant.

3. Dispositif selon la revendication 1 ou 2,
**caractérisé en ce que**
le moyen d'écartement (11) comprend une partie inférieure (17) susceptible de prendre appui sur le plateau tibial, une partie supérieure (19) guidée en déplacement sur la partie inférieure (17), et un organe d'actionnement (21) qui est maintenu en déplacement sur la partie inférieure (17) et qui coopère avec la partie inférieure (17) et avec la partie supérieure (19) de telle sorte qu'un mouvement de réglage de l'organe d'actionnement (21) est converti en un mouvement d'écartement de la partie supérieure (19) en éloignement de la partie inférieure (17).

4. Dispositif selon la revendication 3,
**caractérisé en ce que**
la partie supérieure (19) est susceptible d'être sollicitée depuis le bas par l'organe d'actionnement (21) et d'être poussée en éloignement de la partie inférieure (17) dans la direction d'écartement.

5. Dispositif selon la revendication 3 ou 4,
**caractérisé en ce que**
l'organe d'actionnement (21) est déplaçable en oblique par rapport à la direction d'écartement, et la partie supérieure (19) est déplaçable par rapport à la partie inférieure (17) parallèlement à la direction d'écartement.

6. Dispositif selon l'une des revendications 3 à 5,
**caractérisé en ce que**
l'organe d'actionnement (21) est pourvu à son extrémité libre d'une surface de pression plane (23) qui s'étend parallèlement à un côté inférieur de la partie supérieure (19), susceptible d'être sollicité au moyen de la surface de pression (23), et en particulier perpendiculairement à la direction d'écartement.

7. Dispositif selon l'une des revendications 3 à 6,
**caractérisé en ce que**
un guidage (25) destiné à la partie supérieure (19) et s'étendant parallèlement à la direction d'écartement, et la zone dans laquelle l'organe d'actionnement (21) attaque la partie supérieure (19) sont décalés l'un par rapport à l'autre perpendiculairement à la direction d'écartement.

8. Dispositif selon l'une des revendications 3 à 7,
**caractérisé en ce que**
la partie inférieure (17) et la partie supérieure (19) comprennent chacune une languette d'écartement (27, 29) en forme de plaque par laquelle la partie inférieure (17) peut prendre appui sur le plateau tibial et la partie supérieure (19) peut être pressée contre le condyle de fémur.

9. Dispositif selon la revendication 8,
**caractérisé en ce que**
l'organe d'actionnement (21) est susceptible d'être enfoncé dans l'intervalle entre les deux languettes d'écartement (27, 29) et contre le côté inférieur de la languette d'écartement (29) de la partie supérieure (19), la partie inférieure (17) comprenant un tronçon de base (31) qui s'étend en oblique par rapport à sa languette d'écartement (27), tronçon dans lequel est réalisé un guidage (25) pour la partie supérieure (19) et dans lequel est maintenu l'organe d'actionnement (21).

10. Dispositif selon l'une des revendications 3 à 9,
**caractérisé en ce que**
l'organe d'actionnement (21) est réalisé sous forme de vis de réglage.

11. Dispositif selon l'une des revendications précédentes,
**caractérisé en ce que**
le moyen d'écartement (11) est pourvu d'un moyen d'affichage (33) prévu en particulier sous la forme d'une échelle à partir de laquelle on peut lire une hauteur de consigne de la garniture fonctionnelle (13) sur le moyen d'écartement (11), hauteur qui dépend de l'épaisseur de l'implant tibial à mettre en place.

12. Dispositif selon l'une des revendications précédentes,
**caractérisé en ce que**
la garniture fonctionnelle (13) est réglable par rapport au moyen d'écartement (11) entre des positions discrètes qui sont espacées en correspondance des épaisseurs d'un lot d'implants tibiaux de différentes épaisseurs.

13. Dispositif selon l'une des revendications précédentes,
**caractérisé en ce que**
dans l'état agencé entre le plateau tibial et le condyle de fémur, le moyen d'écartement (11) est mobile par rapport au genou, en particulier en translation sensiblement perpendiculairement à la direction d'écartement et/ou en rotation autour d'un axe sensiblement parallèle à la direction d'écartement.

14. Dispositif selon l'une des revendications précédentes,
**caractérisé en ce que**
le moyen d'écartement (11) est susceptible d'être couplé à plusieurs garnitures fonctionnelles (13) réalisées différemment.

15. Dispositif selon l'une des revendications précédentes,
**caractérisé en ce que**
au moins une garniture fonctionnelle (13) est réalisée sous la forme d'un gabarit de coupe (35) pour déterminer une coupe de condyle (39) à exécuter lorsque le genou est en extension, coupe qui s'étend en particulier sensiblement parallèlement au plateau tibial.

16. Dispositif selon l'une des revendications précédentes,
**caractérisé en ce que**
au moins une garniture fonctionnelle (13) est réalisée sous la forme d'un gabarit de coupe (37) pour déterminer une coupe de condyle (39) à exécuter lorsque le genou est en flexion, coupe qui s'étend en particulier sensiblement parallèlement au plateau tibial.

17. Dispositif selon l'une des revendications précédentes,
**caractérisé en ce que**
au moins une garniture fonctionnelle (13) est réalisée sous la forme d'un gabarit de perçage (41) pour déterminer au moins un perçage de condyle servant à fixer un implant fémoral et à exécuter lorsque le genou est en flexion, perçage qui s'étend en particulier sensiblement parallèlement au plateau tibial.

18. Dispositif selon l'une des revendications précédentes,
**caractérisé en ce que**
au moins une garniture fonctionnelle (13) qui est réalisée en particulier sous forme de gabarit de coupe et de perçage combiné est susceptible d'être fixée sur le condyle de fémur.

19. Dispositif selon l'une des revendications précédentes,
**caractérisé en ce que**
au moins une garniture fonctionnelle (13) comprend un tronçon de corps (47) qui est susceptible d'être couplé au moyen d'écartement (11) et qui s'étend en oblique par rapport à la direction d'écartement, dans l'état couplé au moyen d'écartement (11), et qui est pourvu d'un organe d'immobilisation (49), et un tronçon de tête (51) qui est fermement relié au tronçon de corps (47) et qui est réalisé sous forme de gabarit de coupe et/ou de perçage (35) ou de support pour un gabarit de coupe et/ou de perçage séparé (45).

20. Dispositif selon la revendication 19,
**caractérisé en ce que**
le tronçon de tête (51) est réalisé sous la forme d'un gabarit de coupe (35) pourvu d'une fente (53) destinée à un outil de coupe (55), définissant un plan de coupe et s'étendant perpendiculairement à la direction d'écartement dans l'état couplé au moyen d'écartement (11).

21. Dispositif selon la revendication 19,
**caractérisé en ce que**
le tronçon de tête (51) est réalisé sous forme de support pour le gabarit de coupe et de perçage combiné séparé (45) qui est susceptible d'être relié de façon détachable au tronçon de tête (51) et d'être fixé au condyle de fémur, le genou étant en flexion.

22. Dispositif selon la revendication 21,
**caractérisé en ce que**
le gabarit de coupe et de perçage combiné séparé (45) est déplaçable le long du tronçon de tête (51), dans l'état relié au tronçon de tête (51).

23. Dispositif selon l'une des revendications précédentes,
**caractérisé en ce que**
il est prévu un gabarit de coupe supplémentaire, et **en ce qu'**au moins une garniture fonctionnelle (13) réalisée en particulier sous la forme d'un gabarit de coupe et de perçage combiné est susceptible d'être couplée au gabarit de coupe supplémentaire qui est réalisé pour déterminer au moins une autre coupe de condyle en particulier lorsque le genou est en flexion, l'autre coupe de condyle s'étendant de façon incurvée entre deux surfaces de couple planes (39) réalisées auparavant sur le condyle de fémur.
